Europäisches Patentamt
European Patent Office
Office européen des brevets

(19)

(11) Publication number: **0 095 329**
**A2**

(12) # EUROPEAN PATENT APPLICATION

(21) Application number: 83302859.0

(22) Date of filing: 19.05.83

(51) Int. Cl.$^3$: C 07 D 501/46
A 61 K 31/545
//C07D277/20

(30) Priority: 20.05.82 GB 8214774

(43) Date of publication of application: 30.11.83 Bulletin 83/48

(84) Designated Contracting States: AT BE CH DE FR GB IT LI LU NL SE

(71) Applicant: GLAXO GROUP LIMITED
Clarges House 6-12 Clarges Street
London W1Y 8DH(GB)

(72) Inventor: Looker, Brian Edgar
28 Middleton Avenue
Greenford Middlesex(GB)

(74) Representative: Holmes, Michael John et al,
Frank B. Dehn & Co. European Patent Attorneys Imperial House 15-19 Kingsway
London, WC2B 6UZ,(GB)

(54) **Cephalosporin antibiotics.**

(57) Cephalosporin antibiotics of general formula

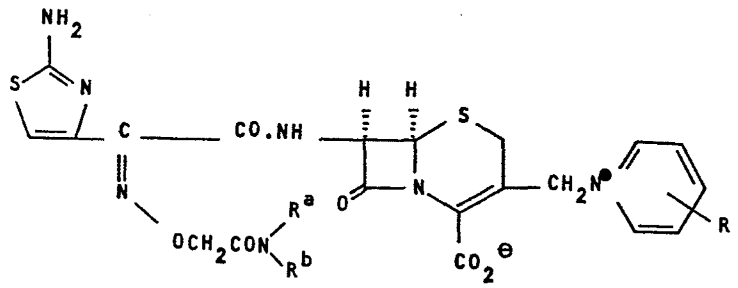

(I)

(wherein $R^a$ and $R^b$, which may be the same or different, each represents a hydrogen atom or a methyl or ethyl group, and R represents a 3- or 4-carbamoyl group or a hydrogen atom) and non-toxic salts and non-toxic metabolically labile esters thereof. The compounds exhibit broad spectrum antibiotic activity both *in vitro* and *in vivo*, and may be used for treating a variety of diseases caused by pathogenic bacteria in human beings and animals. Pharmaceutical compositions containing the compounds and processes for the preparation of the compounds are also described.

EP 0 095 329 A2

Croydon Printing Company Ltd.

"Cephalosporin Antibiotics"

This invention relates to improvements in or relating to cephalosporins. More particularly it relates to new cephalosporin compounds and derivatives thereof having valuable antibiotic activity.

The cephalosporin compounds in this specification are named with reference to "cepham" after J.Amer.Chem. Soc., 1962, 84, 3400, the term "cephem" referring to the basic cepham structure with one double bond.

Cephalosporin antibiotics are widely used in the treatment of diseases caused by pathogenic bacteria in human beings and animals, and are especially useful in the treatment of diseases caused by bacteria which are resistant to other antibiotics such as penicillin compounds, and in the treatment of penicillin-sensitive patients. In many instances it is desirable to employ a cephalosporin antibiotic which exhibits activity against both Gram-positive and Gram-negative micro-organisms, and a significant amount of research has been directed to the development of various types of broad spectrum cephalosporin antibiotics.

Thus, for example in our British Patent Specification No. 1,399,086, we describe a novel class of cephalosporin antibiotics containing a 7β-(α-etherified oximino)-acylamido group, the oximino group having the *syn* configuration. This class of antibiotic compounds is characterised by high antibacterial activity against a range of Gram-positive and Gram-negative organisms coupled with particularly high stability to β-lactamases produced by various Gram-negative organisms.

The discovery of this class of compounds has stimulated further research in the same area in attempts to find compounds which have improved properties, for example against particular classes of organisms especially Gram-negative organisms.

Thus, for example, our British Patent Specification No. 1,555,471 describes cephalosporin antibiotics containing <u>inter alia</u>, a 7β-acylamido side chain of the formula

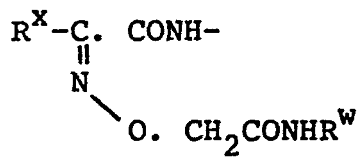

wherein $R^x$ represents a phenyl, thienyl or furyl group and $R^w$ is hydrogen or a $C_{1-4}$ alkyl group and a 3-position substituent which is selected from a wide variety of groups including an optionally substituted pyridiniummethyl group.

In British Patent Specification No. 1,604,971, cephalosporin antibiotics having a wide range of 7β- and 3-position side chains are disclosed. Some of the 7β- possibilities include side-chains of the formula

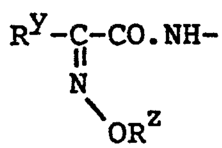

wherein $R^y$ represents <u>inter alia</u> a 2-aminothiazol-4-yl group and $R^z$ represents an alkyl group substituted by a carbamoyl group, and amongst the wide range of 3-position substituents that are allowed for is an optionally substituted pyridiniummethyl group.

Also, in British Patent Specification No. 1,605,175, cephalosporin antibiotics having <u>inter alia</u> a 2-(2-aminothiazol-4-yl)-2-carbamoylmethoxyiminoacetamido group at the 7β-position are disclosed although the 3-position is restricted only to acetoxymethyl. The same 7β-position group is mentioned in European Patent Application No. 7633 although the 3-position is restricted to hydrogen and halogen.

We have now discovered that by the selection of certain 2-(2-aminothiazol-4-yl)-2-(<u>syn</u>)-(optionally

substituted carbamoyl)methoxyimino-acetamido groups at the 7β-position in combination with either a pyridiniummethyl or a 3- or 4-carbamoylpyridiniummethyl group at the 3-position, cephalosporin compounds having particularly advantageous activity (described in more detail below) against a wide range of commonly encountered pathogenic organisms may be obtained.

Accordingly, we provide cephalosporin antibiotics of the general formula (I)

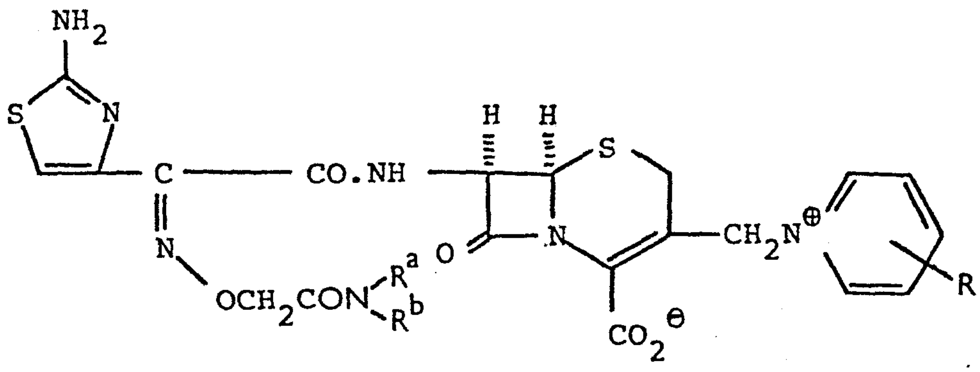

(I)

wherein $R^a$ and $R^b$, which may be the same or different, each represents a hydrogen atom or a methyl or ethyl group, and R represents a 3- or 4-carbamoyl group or a hydrogen atom, and non-toxic salts and non-toxic metabolically labile esters thereof.

Preferred compounds according to the invention are those in which $R^a$ and $R^b$, which may be the same or different, each represents a hydrogen atom or a methyl group.

The compounds according to the invention are <u>syn</u> isomers. The <u>syn</u> isomeric form is defined by the configuration of the group

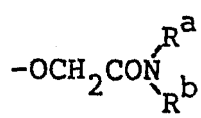

with respect to the carboxamido group. In this Specification, the <u>syn</u> configuration is denoted structurally as

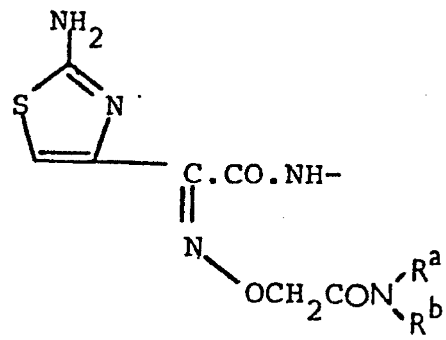

It will be understood that since the compounds according to the invention are geometric isomers, some admixture with the corresponding <u>anti</u> isomer may occur.

The invention also includes within its scope the solvates (especially the hydrates) of the compounds of formula (I) and the solvates of the non-toxic salts and metabolically labile esters of the compounds of formula (I). It also includes within its scope non-toxic salts of the metabolically labile esters of compounds of formula (I) and solvates thereof. It will be appreciated that the solvates should be pharmacologically acceptable.

The compounds according to the present invention may exist in tautomeric forms (for example in respect of the 2-aminothiazolyl group) and it will be understood that such tautomeric forms, e.g. the 2-iminothiazolinyl form, are included within the scope of the invention.

The compounds according to the invention exhibit broad spectrum antibiotic activity both *in vitro* and *in vivo*. They have high activity against both Gram-positive and Gram-negative organisms, including many β-lactamase producing strains. The compounds also possess high stability to β-lactamases produced by a range of Gram-negative and Gram-positive organisms.

Compounds according to the invention have been found to exhibit high activity against strains of *Staphylococcus aureus* and *Staphylococcus epidermidis*,

including penicillinase producing strains of these Gram-positive organisms. This is coupled with high activity against various members of the Enterobacteriaceae (e.g. strains of Escherichia coli, Klebsiella pneumoniae, Citrobacter diversus, Enterobacter cloacae, Serratia marcescens, Proteus mirabilis and indole-positive Proteus organisms such as Proteus vulgaris, Proteus morganii and Providence species), strains of Haemophilus influenzae, and Acinetobacter calcoaceticus as well as good activity against Pseudomonas species.

Non-toxic salt derivatives which may be formed by reaction of the carboxyl group present in the compounds of formula (I) include inorganic base salts such as alkali metal salts (e.g. sodium and potassium salts) and alkaline earth metal salts (e.g. calcium salts); amino acid salts (e.g. lysine and arginine salts); organic base salts (e.g. procaine, phenylethylbenzylamine, dibenzylethylenediamine, ethanolamine, diethanolamine and N-methylglucosamine salts). Other non-toxic salt derivatives include acid addition salts, e.g. formed with hydrochloric, hydrobromic, sulphuric, nitric, phosphoric, formic and trifluoroacetic acids. The salts may also be in the form of resinates formed with, for example, a polystyrene resin or cross-linked polystyrene divinylbenzene copolymer resin containing amino or quaternary amino groups or sulphonic acid groups, or with a resin containing carboxyl groups, e.g. a polyacrylic acid resin. Soluble base salts (e.g. alkali metal salts such as the sodium salt) of the compounds of formula (I) may be used in therapeutic applications because of the rapid distribution of such salts in the body upon administration. Where, however, insoluble salts of compounds (I) are desired in a particular application, e.g. for use in depot preparations, such salts may be formed in conventional manner, for example with appropriate organic amines.

These and other salt derivatives such as the salts with toluene-p-sulphonic and methanesulphonic

acids may be employed as intermediates in the preparation and/or purification of the present compounds of formula (I), for example in the processes described below.

Non-toxic metabolically labile ester derivatives which may be formed by esterification of the carboxyl group in the parent compound of formula (I) include acyloxyalkyl esters, e.g. lower alkanoyloxy-methyl or -ethyl esters such as acetoxy-methyl or -ethyl or pivaloyloxymethyl esters, and alkoxycarbonyloxyalkyl esters e.g. lower alkoxycarbonyloxyethyl esters such as the ethoxycarbonyloxyethyl ester. In addition to the above ester derivatives, the present invention includes within its scope the compounds of formula (I) in the form of other physiologically acceptable equivalents, i.e. physiologically acceptable compounds which, like the metabolically labile esters, are converted in vivo into the parent antibiotic compound of formula (I).

It will be appreciated that the compounds of the invention are usually present in the form of a betaine containing a positively-charged pyridinium group and a carboxylate group, and therefore esters and salts of compounds of formula (I) with bases will be associated with an anion $A^{\ominus}$ to balance the positive charge on the pyridinium ring. Such an anion will also be non-toxic and may be derived from any of the acids described above which will form non-toxic salt derivatives.

Preferred compounds according to the invention by virtue of their high antibiotic activity are those compounds of formula (I) above wherein R represents hydrogen, i.e. compounds of the formula:-

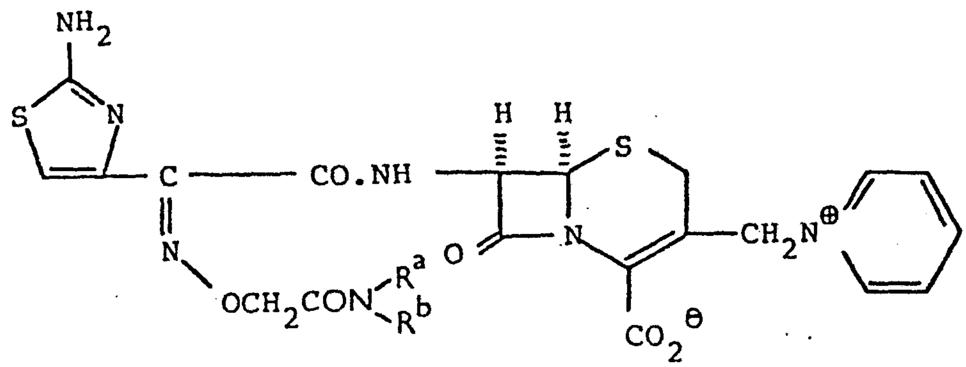

(Ia)

(wherein $R^a$ and $R^b$ are as defined above) and their non-toxic salts and non-toxic metabolically labile esters.

Especially preferred compounds of the invention are those of formula (Ia) in which $R^a$ and $R^b$ both represent hydrogen atoms or methyl groups, or one of $R^a$ and $R^b$ represents a hydrogen atom and the other is a methyl group, as well as the non-toxic salts and non-toxic metabolically labile esters thereof.

The compounds of the invention may be used for treating a variety of diseases caused by pathogenic bacteria in human beings and animals, such as respiratory tract infections and urinary tract infections.

According to another embodiment of the invention we provide a process for the preparation of an antibiotic compound of general formula (I) as hereinbefore defined or a non-toxic salt or non-toxic metabolically labile ester thereof which comprises

(A) acylating a compound of the formula

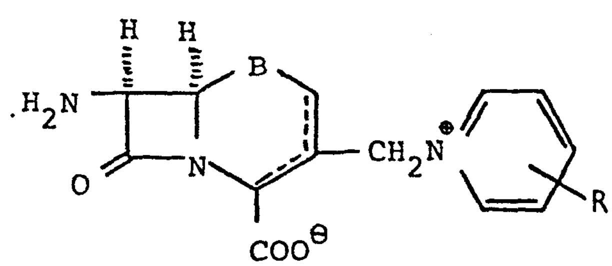

(II)

[wherein R is as defined above; B is $>S$ or $>S \rightarrow O$ (α- or β-); and the dotted line bridging the 2-, 3-, and 4-positions indicates that the compound is a ceph-2-em or ceph-3-em compound] or a salt, e.g. an acid addition salt (formed with, for example, a mineral acid such as hydrochloric, hydrobromic, sulphuric, nitric or phosphoric acid or an organic acid such as methanesulphonic or toluene-p-sulphonic acid) or an N-silyl derivative thereof, or a corresponding compound having a group of formula $-COOR^1$ at the 4-position [where $R^1$ is a hydrogen atom or a carboxyl blocking group, e.g. the residue of an ester-forming aliphatic or araliphatic alcohol or an ester-forming phenol, silanol or stannanol (the said alcohol, phenol, silanol or stannanol preferably containing 1-20 carbon atoms)] and having an associated anion $E^{\ominus}$ such as a halide, e.g. chloride or bromide, or trifluoroacetate anion, with an acid of formula

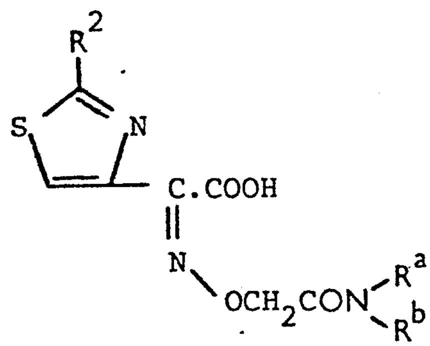

(III)

(wherein $R^a$ and $R^b$ are as defined above, and $R^2$ is an amino or protected amino group) or with an acylating agent corresponding thereto;

(B) reacting a compound of formula

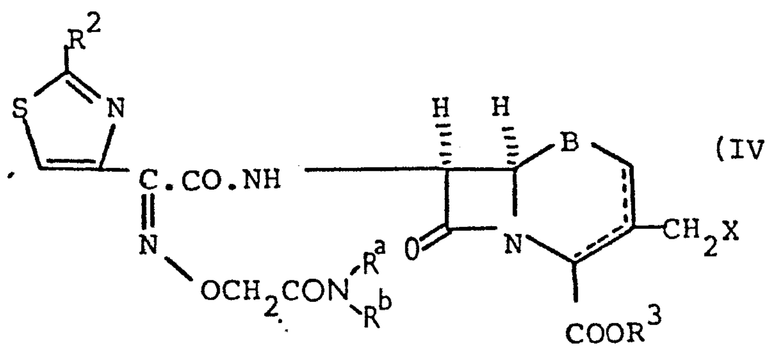

(IV)

(wherein $R^a$, $R^b$, $R^2$, B and the dotted line are as hereinbefore defined; $R^3$ represents hydrogen or a carboxyl blocking group; and X is a replaceable residue of a nucleophile, e.g. an acetoxy or dichloroacetoxy group or a halogen atom such as chlorine, bromine or iodine) or a salt thereof, with a pyridine compound of the formula

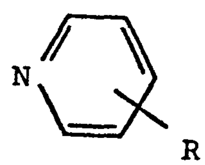

(V)

(wherein R is as defined above);

or (C) reacting a compound of formula

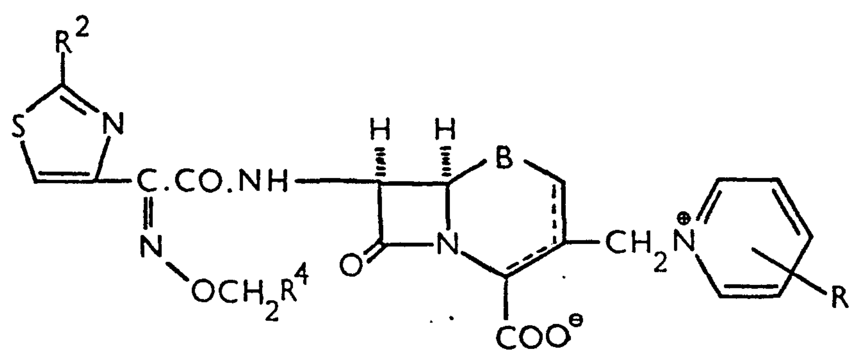

(VI)

(wherein R, $R^2$, B and the dotted line are as defined above, and $R^4$ represents a carboxyl group or an activated derivative thereof) or a corresponding compound having a group of formula $-COOR^1$ at the 4-position (wherein $R^1$ is as defined above) and having an associated anion $E^\ominus$ such as a halide e.g. chloride or bromide or trifluoroacetate anion, with a compound of formula

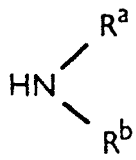

(VII)

(wherein $R^a$ and $R^b$ are as defined above); whereafter, if necessary and/or desired in each instance, any of the following reactions, in any appropriate sequence, are carried out:-

i) conversion of a $\Delta^2$-isomer into the desired $\Delta^3$-isomer,

ii) reduction of a compound wherein B is $>S \rightarrow O$ to form a compound wherein B is $>S$.

iii) conversion of a carboxyl group into a non-toxic metabolically labile ester function;

iv) formation of a non-toxic salt function; and

v) removal of any carboxyl blocking and/or N-protecting groups.

The above reactions i) to v) may be carried out in conventional manner.

In the above-described process (A), the starting material of formula (II) is preferably a compound wherein B is $>S$ and the dotted line represents a ceph-3-em compound. One such starting material which has been found to be particularly suitable for use in process (A) is (6R,7R)-7-amino-3-(1-pyridiniummethyl)-ceph-3-em-4-carboxylate dihydrochloride on account of the high purity in which it can be prepared.

Acylating agents which may be employed in the preparation of compounds of formula (I) include acid halides, particularly acid chlorides or bromides. Such acylating agents may be prepared by reacting an acid (III) or a salt thereof with a halogenating agent e.g. phosphorus pentachloride, thionyl chloride or oxalyl chloride.

Acylations employing acid halides may be effected in aqueous and non-aqueous reaction media, conveniently at temperatures of from -50 to +50°C, preferably -40

to +30°C, if desired in the presence of an acid binding agent. Suitable reaction media include aqueous ketones such as aqueous acetone, aqueous alcohols such as aqueous ethanol, esters such as ethyl acetate, halogenated hydrocarbons such as methylene chloride, amides such as dimethylacetamide, nitriles such as acetonitrile, or mixtures of two or more such solvents. Suitable acid binding agents include tertiary amines (e.g. triethylamine or dimethylaniline), inorganic bases (e.g. calcium carbonate or sodium bicarbonate), and oxiranes such as lower 1,2-alkylene oxides (e.g. ethylene oxide or propylene oxide) which bind hydrogen halide liberated in the acylation reaction.

Acids of formula (III) may themselves be used as acylating agents in the preparation of compounds of formula (I). Acylations employing acids (III) are desirably conducted in the presence of a condensing agent, for example a carbodiimide such as N,N'-dicyclohexylcarbodiimide or N-ethyl-N'-$\gamma$-dimethylaminopropylcarbodiimide; a carbonyl compound such as carbonyldiimidazole; or an isoxazolium salt such as N-ethyl-5-phenylisoxazolium perchlorate.

Acylation may also be effected with other amide-forming derivatives of acids of formula (III) such as, for example, an activated ester, a symmetrical anhydride or a mixed anhydride (e.g. formed with pivalic acid or with a haloformate, such as a lower alkylhaloformate). Mixed anhydrides may also be formed with phosphorus acids (for example phosphoric or phosphorous acids), sulphuric acid or aliphatic or aromatic sulphonic acids (for example toluene-p-sulphonic acid). An activated ester may conveniently be formed <u>in situ</u> using, for example, 1-hydroxybenzotriazole in the presence of a condensing agent as set out above. Alternatively, the activated ester may be preformed.

Acylation reactions involving the free acids or their above-mentioned amide-forming derivatives are desirably effected in an anhydrous reaction medium,

e.g. methylene chloride, tetrahydrofuran, dimethylformamide or acetonitrile.

An alternative method of activation is, for example, by reacting the acid of formula (III) with a solution or suspension preformed by adding a carbonyl halide, in particular oxalyl chloride or phosgene, or a phosphoryl halide such as phosphorus oxychloride to a solvent such as a halogenated hydrocarbon, for example methylene chloride, containing a lower acyl tertiary amide such as N,N-dimethylformamide. The activated form of the the acid of formula (III) may then be reacted with the 7-amino compound of formula (II) in a suitable solvent or mixture of solvents for example an alkanol such as an alcohol, e.g. aqueous ethanol or aqueous industrial methylated spirts. The acylation reaction may conveniently be effected at temperatures of from -50° to +50°C, preferably -40° to +30°C, if desired in the presence of an acid binding agent, for example as described above (e.g. triethylamine).

If desired, the above acylation reactions may be carried out in the presence of a catalyst such as 4-dimethylaminopyridine.

The acids of formula (III) and acylating agents corresponding thereto may, if desired, be prepared and employed in the form of their acid addition salts. Thus, for example, acid chlorides may conveniently be employed as their hydrochloride salts, and acid bromides as their hydrobromide salts.

The pyridine compound of formula (V) may act as a nucleophile to displace a wide variety of substituents X from the cephalosporin of formula (IV). To some extent the facility of the displacement is related to the $pK_a$ of the acid HX from which the substituent is derived. Thus atoms or groups X derived from strong acids tend, in general, to be more easily displaced than atoms or groups derived from weaker acids. The facility of the displacement is also related, to

some extent, to the precise character of the substituent R in the compound of formula (V).

The displacement of X by the pyridine compound of formula (V) may conveniently be effected by maintaining the reactants in solution or suspension. The reaction is advantageously effected using from 1 to 10 moles of the pyridine compound.

Nucleophilic displacement reactions may conveniently be carried out on those compounds of formula (IV) wherein the substituent X is a halogen atom or an acyloxy group, for example as discussed below.

Acyloxy groups

Compounds of formula (IV) wherein X is an acetoxy group are convenient starting materials for use in the nucleophilic displacement reaction with the pyridine compound of formula (V). Alternative starting materials in this class include compounds of formula (IV) in which X is the residue of a substituted acetic acid e.g. chloroacetic acid, dichloroacetic acid and trifluoroacetic acid.

Displacement reactions on compounds (IV) possessing X substituents of this class, particularly in the case where X is an acetoxy group, may be facilitated by the presence in the reaction medium of iodide or thiocyanate ions. Reactions of this type are described in more detail in British Patent Specifications Nos. 1,132,621 and 1,171,603.

The substituent X may also be derived from formic acid, a haloformic acid such as chloroformic acid, or a carbamic acid.

When using a compound of formula (IV) in which X represents an acetoxy or substituted acetoxy group, it is generally desirable that the group $R^3$ in formula (IV) should be a hydrogen atom and that B should represent >S. In this case, the reaction is advantageously effected in an aqueous medium, preferably at a pH of 5 to 8, particularly 5.5 to 7.

The above-described process employing compounds of formula (IV) in which X is the residue of a substituted acetic acid may be carried out as described in British Patent Specification No. 1,241,657.

When using compounds of formula (IV) in which X is an acetoxy group, the reaction is conveniently effected at a temperature of 30°C to 110°C, preferably 50° to 80°C.

Halogens

Compounds of formula (IV) in which X is a chlorine, bromine or iodine atom can also be conveniently used as starting materials in the nucleophilic displacement reaction with the pyridine compound of formula (V). When using compounds of formula (IV) in this class, B may represent $>S \longrightarrow O$ and $R^3$ may represent a carboxyl blocking group. The reaction is conveniently effected in a non-aqueous medium which preferably comprises one or more organic solvents, advantageously of a polar nature such as ethers, e.g. dioxan or tetrahydrofuran, esters, e.g. ethyl acetate, amides, e.g. formamide and N,N-dimethylformamide, and ketones e.g. acetone. In certain cases the pyridine compound itself may be the solvent. Other suitable organic solvents are described in more detail in British Patent Specification No. 1,326,531. The reaction medium should be neither extremely acidic nor extremely basic. In the case of reactions carried out on compounds of formula (IV) in which $R^3$ is a carboxyl blocking group the 3-pyridiniummethyl product will be formed as the corresponding halide salt which may, if desired, be subjected to one or more ion exchange reactions to obtain a salt having the desired anion.

When using compounds of formula (IV) in which X is a halogen atom as described above, the reaction is conveniently effected at a temperature of -10° to +50°, preferably +10° to +30°C.

In process (C) above, compounds of formula (VI) having at the 4-position a group of formula $-COOR^1$

wherein $R^1$ is a carboxyl blocking group are preferably employed. Where the group $R^4$ represents a carboxyl group the reaction between the compound of formula (VI) and the compound of formula (VII) is desirably conducted in the presence of a coupling agent as described above for process (A), such as N,N'-dicyclohexylcarbodiimide. Where $R^4$ represents an activated carboxyl group this may be for example an acid halide e.g. chloride, or a carboxylic ester group, preferably a lower alkyl ester e.g. methyl. When the compound of formula (VII) is ammonia this may conveniently be gaseous ammonia or a solution of ammonia in water. Suitable reaction media include ketones e.g. acetone, alcohols e.g. methanol or ethanol and esters e.g. ethyl acetate. The reaction is conveniently effected at temperatures of from -20 to +40°C.

The reaction product may be separated from the reaction mixture, which may contain, for example, unchanged cephalosporin starting material and other substances, by a variety of processes including recrystallisation, ionophoresis, column chromatography and use of ion-exchangers (for example by chromatography on ion-exchange resins) or macroreticular resins.

A $\Delta^2$-cephalosporin ester derivative obtained in accordance with the process of the invention may be converted into the corresponding desired $\Delta^3$-derivative by, for example, treatment of the $\Delta^2$-ester with a base, such as pyridine or triethylamine.

A ceph-2-em reaction product may also be oxidised to yield the corresponding ceph-3-em 1-oxide, for example by reaction with a peracid, e.g. peracetic or *m*-chloroperbenzoic acid; the resulting sulphoxide may subsequently be reduced as described hereinafter to yield the corresponding desired ceph-3-em sulphide.

Where a compound is obtained in which B is $>S \rightarrow O$ this may be converted into the corresponding sulphide by, for example, reduction of the corresponding acyloxysulphonium or alkoxysulphonium salt prepared *in situ*

by reaction with e.g. acetyl chloride in the case of an acetoxysulphonium salt, reduction being effected by, for example, sodium dithionite or by iodide ion as in a solution of potassium iodide in a water-miscible solvent e.g. acetic acid, acetone, tetrahydrofuran, dioxan, dimethylformamide or dimethylacetamide. The reaction may be effected at a temperature of from -20° to +50°C.

Metabolically labile ester derivatives of the compounds of formula (I) may be prepared by reacting a compound of formula (I) or a salt or protected derivative thereof with the appropriate esterifying agent such as an acyloxyalkyl halide or alkoxycarbonyloxyalkyl halide (e.g. iodide) conveniently in an inert organic solvent such as dimethylformamide or acetone, followed, where necessary, by removal of any protecting groups.

Base salts of the compounds of formula (I) may be formed by reacting an acid of formula (I) with an appropriate base. Thus, for example, sodium or potassium salts may be prepared using the respective 2-ethylhexanoate or hydrogen carbonate salt. Acid addition salts may be prepared by reacting a compound of formula (I) or a metabolically labile ester derivative thereof with the appropriate acid.

Where a compound of formula (I) is obtained as a mixture of isomers, the syn isomer may be obtained by, for example, conventional methods such as crystallisation or chromatography.

For use as starting materials for the preparation of compounds of general formula (I) according to the invention, compounds of general formula (III) and amide-forming derivatives e.g. acid halides and anhydrides corresponding thereto in their syn isomeric form or in the form of mixtures of the syn isomers and the corresponding anti isomers containing at least 90% of the syn isomer are preferably used.

Acids of formula (III) may be prepared for example

as described in British Patent Specification Nos. 1,605,176 or 1,605,177, or European Patent Application Specification No. 7633.

Where X is a halogen (i.e. chlorine, bromine or iodine) atom in formula (IV), ceph-3-em starting compounds may be prepared in conventional manner, e.g. by halogenation of a 7β-protected amino-3-methyl-ceph-3-em-4-carboxylic acid ester 1β-oxide, removal of the 7β-protecting group, acylation of the resulting 7β-amino compound to form the desired 7β-acylamido group, e.g. in an analogous manner to process (A) above, followed by reduction of the 1β-oxide group later in the sequence. This is described in British Patent No. 1,326,531. The corresponding ceph-2-em compounds may be prepared by the method of Dutch published Patent Application No. 6,902,013 by reaction of a 3-methylceph-2-em compound with N-bromosuccinimide to yield the corresponding 3-bromomethylceph-2-em-compound.

Where X in formula (IV) is an acetoxy group, such starting materials may be prepared for example by acylation of 7-aminocephalosporanic acid, e.g. in an analogous manner to process (A) above. Compounds of formula (IV) in which X represents other acyloxy groups can be prepared by acylation of the corresponding 3-hydroxymethyl compounds which may be prepared for example by hydrolysis of the appropriate 3-acetoxymethyl compounds, e.g. as described for example in British Patent Specifications Nos. 1,474,519 and 1,531,212.

The starting materials of formula (II) may also be prepared in conventional manner, for example, by nucleophilic displacement of the corresponding 3-acetoxy-methyl compound with the appropriate nucleophile, e.g. as described in British Patent Specification No. 1,028,563, or by the method described in British Patent Specification No. 2052490A.

A further method for the preparation of the starting materials of formula (II) comprises deprotecting

a corresponding protected 7β-amino compound in conventional manner, e.g. using $PCl_5$.

The starting materials of formula (VI) may be prepared for example by acylation of the corresponding 7-amino cephalosporin, in an analogous manner to process (A) above or by nucleophilic displacement of a corresponding 3-acyloxymethyl or 3-halomethyl cephalosporin with the appropriate nucleophile, in an analogous manner to process (B) above, as described, for example in British Patent Specification No. 2062624A.

It should be appreciated that in some of the above transformations it may be necessary to protect any sensitive groups in the molecule of the compound in question to avoid undesirable side reactions. For example, during any of the reaction sequences referred to above it may be necessary to protect the $NH_2$ group of the aminothiazolyl moiety, for example by tritylation, acylation (e.g. chloroacetylation or formylation), protonation or other conventional method. The protecting group may thereafter be removed in any convenient way which does not cause breakdown of the desired compound, e.g. in the case of a trityl group by using an optionally halogenated carboxylic acid, e.g. acetic acid, formic acid, chloroacetic acid or trifluoroacetic acid or using a mineral acid, e.g. hydrochloric acid or mixtures of such acids, preferably in the presence of a protic solvent such as water, or, in the case of a chloroacetyl group, by treatment with thiourea.

Carboxyl blocking groups used in the preparation of compounds of formula (I) or in the preparation of necessary starting materials are desirably groups which may readily be split off at a suitable stage in the reaction sequence, conveniently at the last stage. It may, however, be convenient in some instances to employ non-toxic metabolically labile carboxyl blocking groups such as acyloxy-methyl or -ethyl groups (e.g. acetoxy-methyl or -ethyl or pivaloyloxymethyl)

or alkoxycarbonyloxyalkyl groups (e.g. ethoxycarbonyloxyethyl) and retain these in the final product to give an appropriate ester derivative of a compound of formula (I).

Suitable carboxyl blocking groups are well known in the art, a list of representative blocked carboxyl groups being included in British Patent No. 1,399,086. Preferred blocked carboxyl groups include aryl lower alkoxycarbonyl groups such as p-methoxybenzyloxycarbonyl, p-nitrobenzyloxycarbonyl and diphenylmethoxycarbonyl; lower alkoxycarbonyl groups such as t-butoxycarbonyl; and lower haloalkoxycarbonyl groups such as 2,2,2-trichloroethoxycarbonyl. The carboxyl blocking group may subsequently be removed by any of the appropriate methods disclosed in the literature; thus, for example, acid or base catalysed hydrolysis is applicable in many cases, as are enzymically-catalysed hydrolyses.

The antibiotic compounds of the invention may be formulated for administration in any convenient way, by analogy with other antibiotics and the invention therefore includes within its scope pharmaceutical compositions comprising an antibiotic compound in accordance with the invention adapted for use in human or veterinary medicine. Such compositions may be presented for use in conventional manner with the aid of any necessary pharmaceutical carriers or excipients.

The antibiotic compounds according to the invention may be formulated for injection and may be presented in unit dose form, in ampoules, or in multi-dose containers, if necessary with an added preservative. The compositions may also take such forms as suspensions, solutions, or emulsions in oily or aqueous vehicles, and may contain formulatory agents such as suspending, stabilising and/or dispersing agents. Alternatively the active ingredient may be in powder form for reconstitution with a suitable vehicle, e.g. sterile, pyrogen-free water, before use.

If desired, such powder formulations may contain an appropriate non-toxic base in order to improve the water-solubility of the active ingredient and/or to ensure that when the powder is reconstituted with water, the pH of the resulting aqueous formulation is physiologically acceptable. Alternatively the base may be present in the water with which the powder is reconstituted. The base may be, for example, an inorganic base such as sodium carbonate, sodium bicarbonate or sodium acetate, or an organic base such as lysine or lysine acetate.

The antibiotic compounds may also be presented in a form suitable for absorption by the gastro-intestinal tract e.g. as tablets or capsules.

The antibiotic compounds may also be formulated as suppositories e.g. containing conventional suppository bases such as cocoa butter or other glycerides.

Compositions for veterinary medicine may, for example, be formulated as intramammary preparations in either long acting or quick-release bases.

The compositions may contain from 0.1% upwards, e.g. 0.1-99% of the active material, depending on the method of administration. When the compositions comprise dosage units, each unit will preferably contain 100-3000 mg e.g. 500-2000 mg of the active ingredient. The dosage for adult human treatment will preferably range from 500 to 12000 mg e.g. 1500-9000 mg per day, depending inter alia on the nature of the infection and the route and frequency of administration. In general, intravenous or intramuscular administration will be employed, for example using 1000 to 3000 mg per day of the active ingredient in adult human treatment. In treating Pseudomonas infections higher daily doses may be required. It will be appreciated that in some circumstances, for example, in the treatment of neonates, smaller dosage units and daily dosages may be desirable.

The antibiotic compounds according to the invention may be administered in combination with other therapeutic agents such as antibiotics, for example penicillins or other cephalosporins.

The following Examples illustrate the invention. All temperatures are in °C. Sorbsil is silica gel, manufactured by Joseph Crosfield and Son of Warrington, Lancashire, England; Amberlite XAD-2 is a non-ionic macroreticular adsorption resin, manufactured by Rohm and Haas of Philadelphia, U.S.A.

Preparation 1

Ethyl (Z)-2-(N-methylcarbamoylmethoxyimino)-2-(2-tritylaminothiazol-4-yl)acetate

Ethyl (Z)-2-carboxymethoxyimino-2-(2-tritylaminothiazol-4-yl)acetate (described in German OLS 2812625) (7.22 g) was dissolved in tetrahydrofuran (56 ml) and methylene chloride (42 ml) and the solution was stirred with cooling to -25°. N-Methylmorpholine (1.54 ml) was added followed by isobutylchloroformate (1.82 ml). After 15 minutes at -25 to -20° the solution was cooled to -35° and methylamine was passed into the reaction mixture for 5 minutes. The mixture was allowed to warm to 21° over 1.5 hours, then it was evaporated to give an oil, which was redissolved in ethanol (ca. 70 ml). The crystals which separated were collected by filtration, washed with ethanol and dried to give the title compound (5.13 g). $\lambda_{max}$ (ethanol) 308.5 nm ($E^{1\%}_{1cm}$ 90); $\lambda_{inf}$ 233 nm ($E^{1\%}_{1cm}$ 410); 272 nm ($E^{1\%}_{1cm}$ 182); 260 nm ($E^{1\%}_{1cm}$ 269) and 266 nm ($E^{1\%}_{1cm}$ 226). $\nu_{max}$ ($CHBr_3$) 3400 (NH), 1730 (ester), 1675 and 1530 $cm^{-1}$ (amide bands).

Preparation 2

(Z)-2-(N-Methylcarbamoylmethoxyimino)-2-(2-tritylaminothiazol-4-yl)acetic acid.

The product of Preparation 1 (5 g) was dissolved in dioxan (32 ml) and 2N sodium hydroxide solution with stirring. After 1 hour a solid separated and the mixture was allowed to stand overnight. The mixture was acidified by addition of 2N hydrochloric acid and partitioned between ethyl acetate and water. The aqueous layer was extracted with more ethyl acetate and the combined organic layers were washed with brine, dried with magnesium sulphate and evaporated to give

the title compound (5.17 g); $\lambda_{max}$ (ethanol) 235 nm ($E^{1\%}_{1cm}$ 365); $\lambda_{inf}$ 259.5 nm($E^{1\%}_{1cm}$ 220), 266 nm ($E^{1\%}_{1cm}$ 205) and 271.5 nm ($E^{1\%}_{1cm}$ 189); $\nu_{max}$ (Nujol) 3600-2200 (NH and COOH); 1730 (COOH) 1660 and 1530 $cm^{-1}$ (amide). The n.m.r. spectrum indicated that the product is associated with some ethyl acetate.

Preparation 3

Ethyl (Z)-2-(N,N-dimethylcarbamoylmethoxyimino)-2-(2-tritylaminothiazol-4-yl)acetate.

Ethyl (Z)-2-carboxymethoxyimino-2-(2-tritylaminothiazol-4-yl)acetate (7.22 g) (described in German OLS 2812625) was dissolved in tetrahydrofuran (56 ml) and methylene chloride (42 ml). N-Methylmorpholine (1.54 ml) was added and the solution was stirred under nitrogen with cooling to -20°, followed by the addition of isobutylchloroformate (1.82 ml). After 15 minutes the solution was cooled to -35° and dimethylamine was passed into the mixture for 5 minutes. The mixture was stirred at -30° for 15 minutes and then at 21° for 1.5 hours. The mixture was evaporated to a gum which was mixed with ethyl acetate. The resulting suspension was filtered through Sorbsil silica gel (100 g) in ethyl acetate to give the title compound (6.33 g). $\lambda_{max}$ (ethanol) 233.5 nm ($E^{1\%}_{1cm}$ 394); $\lambda_{inf}$ 260 nm ($E^{1\%}_{1cm}$ 247), 265 nm ($E^{1\%}_{1cm}$ 206); 271.5 nm ($E^{1\%}_{1cm}$ 163) and 297 nm ($E^{1\%}_{1cm}$ 84). $\nu_{max}$ ($CHBr_3$) 3400 (NH) 1734 (ester) and 1646 $cm^{-1}$ (amide).

Preparation 4

(Z)-2-(N,N-Dimethylcarbamoylmethoxyimino)-2-(2-tritylaminothiazol-4-yl)acetic acid

The product of Preparation 3 (6.08 g) was dissolved in dioxan (39 ml) and 2N sodium hydroxide solution

(11.2 ml) was added with stirring. After 30 minutes a solid separated and the mixture was allowed to stand overnight. The mixture was acidified with 2N hydrochloric acid and partitioned between ethyl acetate and water. The aqueous layer was extracted with more ethyl acetate and the combined organic layers were washed with brine, dried with magnesium sulphate and evaporated to a gum which was triturated with diethyl ether to give the title compound (5.05 g). $\nu_{max}$ (Nujol) 3420 (NH) 1735 (COOH) and 1618 $cm^{-1}$ (amide); $\tau$(DMSO - $d_6$) values include 1.22 (s N$\underline{H}$), 2.70 (s trityl protons), 3.16 (s thiazole proton), 5.36 (s C$\underline{H}_2$) and 7.10 and 7.20 (s,s N,N-dimethyl protons).

Preparation 5

(6R,7R)-3-Acetoxymethyl-7-[(Z)-2-(2-aminothiazol-4-yl)-2-(carbamoylmethoxyimino)acetamido]ceph-3-em-4-carboxylic acid, Hydrochloride salt

t-Butyl (6R,7R)-3-acetoxymethyl-7-[(Z)-2-carbamoylmethoxyimino-2-(2-tritylaminothiazol-4-yl)acetamido]ceph-3-em-4-carboxylate (797 mg) (described in German OLS 2812625) was dissolved in formic acid (3.5 ml) at 21° and concentrated hydrochloric acid (0.27 ml) was added with stirring. After 1 hour the mixture was filtered and the filter cake was leached with formic acid. The combined filtrates were added dropwise to stirred di-isopropyl ether (80 ml). The precipitate which formed was collected by filtration, washed with di-isopropyl ether and dried to give the title compound (570 mg).

$\lambda_{max}$ (pH6 buffer) 233 nm ($E^{1\%}_{1cm}$ 278); $\lambda_{inf}$ 253.5 nm ($E^{1\%}_{1cm}$ 244), 279.5 nm ($E^{1\%}_{1cm}$ 142) and 295 nm ($E^{1\%}_{1cm}$ 105); $\nu_{max}$ (Nujol) 3300 and 3180 (-NH- and $NH_2$), 1780 (β-lactam), 1720 ($-OCOCH_3$ and -COOH), 1674 and 1540 (-CONH-), and 1674 $cm^{-1}$ ($-CONH_2$).

Example 1

(6R,7R)-7-[(Z)-2-(2-Aminothiazol-4-yl)-2-(N-methylcarbamoylmethoxyimino)acetamido]-3-(1-pyridiniummethyl)-ceph-3-em-4-carboxylate, Dihydrochloride salt

Oxalyl chloride (0.28 ml) was added to a stirred solution of dimethylformamide (0.29 ml) in methylene chloride (10 ml) at -20°. The mixture was stirred in an ice-water bath for ten minutes and then recooled to -20°. (Z)-2-(N-Methylcarbamoylmethoxyimino)-2-(2-tritylaminothiazol-4-yl)acetic acid (1.52 g) was added and the solution formed was stirred in an ice-water bath for ten minutes, with cooling to -15°. This solution was added to a solution of (6R,7R)-7-amino-3-(1-pyridiniummethyl)ceph-3-em-4-carboxylate dihydrochloride salt dihydrate (1.14 g) in industrial methylated spirits (9 ml) and water (23 ml) containing triethylamine (1.76 ml) at -7°. The reaction mixture was allowed to warm to 21° over 20 minutes. The solution was swirled with water and the aqueous phase was extracted with methylene chloride. The combined organic solutions were swirled with more water and the organic layer was dried with magnesium sulphate. A solid started to separate. The mixture was filtered, the filtrate was evaporated and the residue was triturated with diethyl ether to give a solid (340 mg). The magnesium sulphate mixed with the solid which had been separated by filtration was leached with water. The residue was triturated with water and diethyl ether to give further solid (900 mg). The two ether triturated solids were combined.

The solids (1.16 g) were dissolved in formic acid (5 ml) and concentrated hydrochloric acid (0.4 ml) was added. After one hour the precipitate was separated by filtration, the filter-cake was leached with formic acid and the combined filtrates were evaporated to

a gum. This gum was triturated with acetone to give the title compound (0.63 g), $[\alpha]_D^{21}$ -31.4° (c 1.05, dimethylsulphoxide), $\lambda_{max}$ (pH6 buffer) 236.5 nm ($E_{1cm}^{1\%}$ 270) and 254.5 nm ($E_{1cm}^{1\%}$ 278), $\lambda_{inf}$ 286 nm ($E_{1cm}^{1\%}$ 124).

## Example 2

(6R,7R)-7-[(Z)-2-(2-Aminothiazol-4-yl)-2-(N,N-dimethylcarbamoylmethoxyimino)acetamido]-3-(1-pyridiniummethyl)ceph-3-em-4-carboxylate, Dihydrochloride salt.

Oxalyl chloride (0.28 ml) was added to a stirred solution of dimethylformamide (0.29 ml) in methylene chloride (10 ml) at -20°. The mixture was stirred in an ice-water bath for ten minutes and then recooled to -20°. (Z)-2-(N,N-Dimethylcarbamoylmethoxyimino)-2-(2-tritylaminothiazol-4-yl)acetic acid (1.55 g) was added and the solution formed was stirred in an ice-water bath for ten minutes. The solution was cooled to -20° and poured into a solution of (6R,7R)-7-amino-3-(1-pyridiniummethyl)ceph-3-em-4-carboxylate dihydrochloride salt dihydrate (1.14 g) in industrial methylated spirits (9 ml) and water (2.25 ml) containing triethylamine (1.76 ml) at -7°. The reaction mixture was allowed to warm to 21° over 15 minutes. The solution was swirled with water and the aqueous phase was extracted with methylene chloride. The combined organic solutions were swirled with more water, the organic layer was dried with magnesium sulphate and evaporated to a gum. This was triturated with diisopropylether to give a solid (2.05 g). This solid (1.95 g) was dissolved in formic acid (8 ml) and concentrated hydrochloric acid (0.68 ml) was added. After one hour the precipitate was separated by filtration, the filter-cake was leached with formic acid and the combined filtrates were evaporated to a gum. This gum was triturated with acetone to give the title compound (1.50 g), $[\alpha]_D^{21}$-45.85° (c 0.87, dimethylsul-

phoxide), $\lambda_{max}$ (pH6 buffer) 235.5 nm ($E^{1\%}_{1cm}$ 249) and 255 nm ($E^{1\%}_{1cm}$ 256), $\lambda_{inf}$ 292.5 nm ($E^{1\%}_{1cm}$ 101).

EXAMPLE 3

(6R,7R)-7-[(Z)-2-(2-Aminothiazol-4-yl)-2-(carbamoylmethoxyimino)acetamido]-3-(1-pyridiniummethyl)ceph-3-em-4-carboxylate

(6R,7R)-3-Acetoxymethyl-7-[(Z)-2-(2-aminothiazol-4-yl)-2-(carbamoylmethoxyimino)acetamido]ceph-3-em-4-carboxylic acid, hydrochloride salt (2.67 g) was dissolved in water (2.5 ml) by warming to 50° and by the addition of sodium bicarbonate (ca. 1.0 g). Pyridine (1 ml) was added to the solution, followed by sodium iodide (10 g). The resulting syrup was heated to 80° for 45 minutes. The mixture was allowed to cool to 21° and then acetone (100 ml) was added. After trituration the precipitate was collected by filtration, washed with acetone and dried to give a solid (2.84 g). This solid was dissolved in water and loaded onto a column of Amberlite XAD-2 resin (300 g) in water. The column was washed with water, then eluted with 25% ethanol in water. Appropriate fractions were combined and evaporated to a gum which was triturated with acetone and dried to give the title compound (870 g).

$\nu_{max}$ (Nujol) 3400, 3300 and 3180 (NH and $NH_2$); 1772 (β-lactam); 1674 and 1532 (-CONH-); 1674 ($-CONH_2$) and 1610 $cm^{-1}$ ($COO^-$). τ($D_2O$) values include 1.06, 1.44 and 1.92 (pyridinium protons); 2.96 (thiazole proton); 4.14 (C7-H); and 4.43 and 4.67 (ABq J 14Hz 3-$CH_2$).

Pharmacy Example

Dry Powder for Injection

Fill sterile (6R,7R)-7-[(Z)-2-(2-aminothiazol-4-yl)-2-(carbamoylmethoxyimino)acetamido]-3-(1-pyridiniummethyl)ceph-3-em-4-carboxylate aseptically into glass vials, the content of each container being equivalent to 1g of the anhydrous, pure material. Purge the vial headspaces with sterile nitrogen. Close the vials using rubber discs or plugs and metal overseals, applied by crimping, to prevent gaseous exchange or ingress of micro-organisms. The product may be constituted by dissolving in Water for Injections or other suitable sterile vehicle shortly before administration.

CLAIMS

1. Cephalosporin antibiotics of general formula

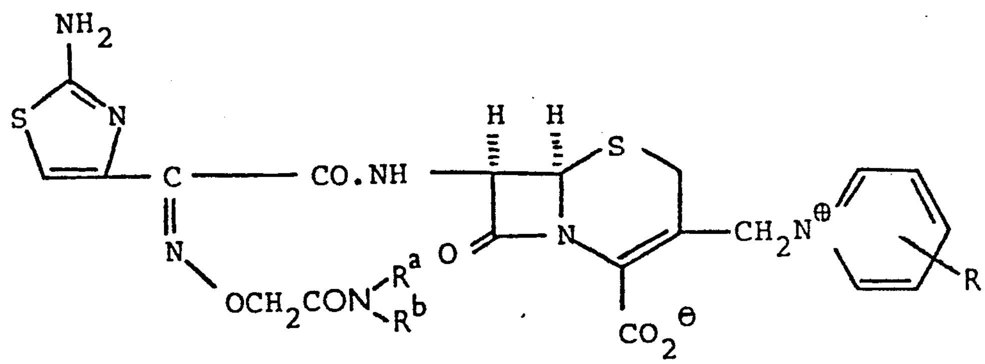

(I)

(wherein $R^a$ and $R^b$, which may be the same or different, each represents a hydrogen atom or a methyl or ethyl group, and R represents a 3- or 4-carbamoyl group or a hydrogen atom) and non-toxic salts and non-toxic metabolically labile esters thereof.

2. A compound according to claim 1 wherein R represents a hydrogen atom.

3. A compound according to either of claims 1 and 2 wherein $R^a$ and $R^b$ both represent hydrogen atoms or methyl groups, or one of $R^a$ and $R^b$ represents a hydrogen atom and the other is a methyl group.

4. A compound according to claim 1 which is:-

(6R,7R)-7-[(Z)-2-(2-aminothiazol-4-yl)-2-(N-methylcarbamoylmethoxyimino)acetamido]-3-(1-pyridiniummethyl)ceph-3-em-4-carboxylate or a non-toxic salt thereof;

(6R,7R)-7-[(Z)-2-(2-aminothiazol-4-yl)-2-(N,N-dimethylcarbamoylmethoxyimino)acetamido]-3-(1-pyridiniummethyl)ceph-3-em-4-carboxylate or a non-toxic salt thereof; or

(6R,7R)-7-[(Z)-2-(2-aminothiazol-4-yl)-2-(carbamoylmethoxyimino)acetamido]-3-(1-pyridiniummethyl)-ceph-3-em-4-carboxylate or a non-toxic salt thereof.

5. A process for the preparation of a compound according to claim 1 which comprises

(A) acylating a compound of formula

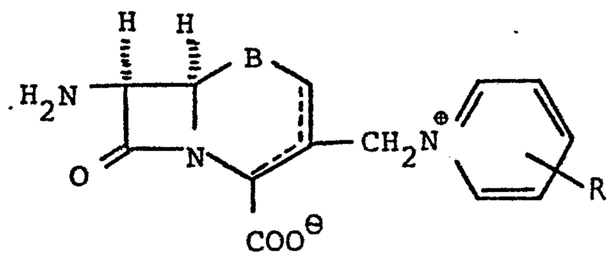

(II)

(wherein R is as defined in claim 1; B is $>S$ or $>S \rightarrow O$; and the dotted line bridging the 2-, 3- and 4- positions indicates that the compound is a ceph-2-em or ceph-3-em compound) or a salt or N-silyl derivative thereof, or a corresponding compound having a group $-COOR^1$ at the 4-position (where $R^1$ is a hydrogen atom or a carboxyl blocking group) and having an associated anion $E^{\ominus}$, with an acid of formula

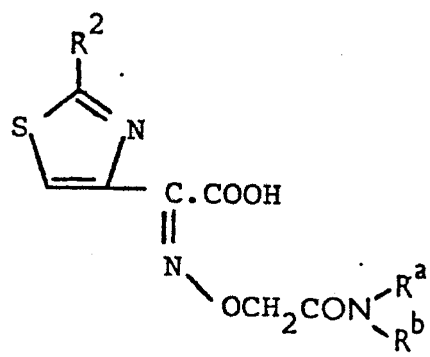

(III)

(wherein $R^a$ and $R^b$ are as defined in claim 1, and $R^2$ is an amino or protected amino group) or with an acylating agent corresponding thereto;

(B) reacting a compound of formula

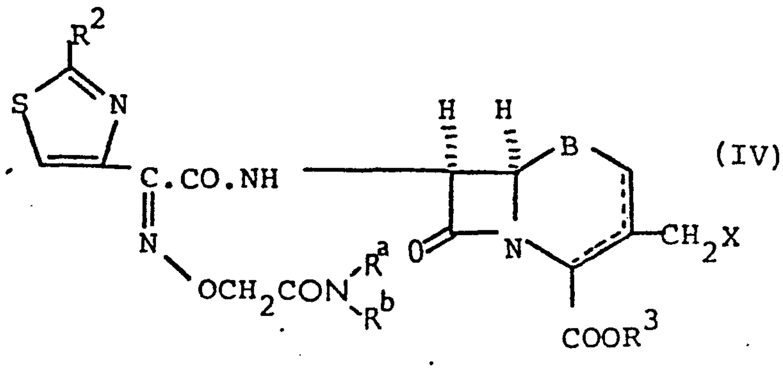

(IV)

(wherein $R^a$, $R^b$, $R^2$, B and the dotted line are as defined above; $R^3$ represents hydrogen or a carboxyl blocking group; and X is a replaceable residue of a nucleophile) or a salt thereof, with a pyridine compound of formula

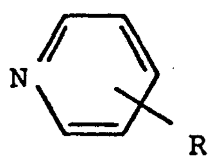

(V)

(wherein R is as defined above); or

(C) reacting a compound of formula

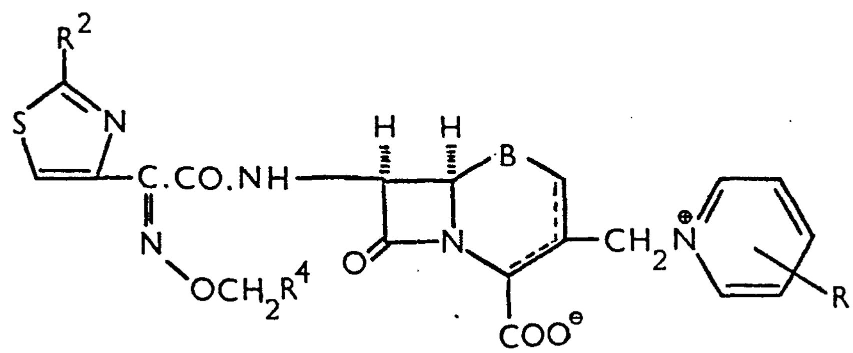

(VI)

(wherein R, $R^2$, $R^3$, B and the dotted line are as defined above, and $R^4$ represents a carboxyl group or an activated derivative thereof) or a corresponding compound having

a group of formula $-COOR^1$ at the 4-position (wherein $R^1$ is as defined above) and having an associated anion $E^{\ominus}$, with a compound of formula

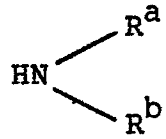 (VII)

(wherein $R^a$ and $R^b$ are as defined above); whereafter, if necessary and/or desired in each instance, any of the following reactions, in any appropriate sequence are carried out:-

i) conversion of a $\Delta^2$-isomer into the desired $\Delta^3$-isomer,

ii) reduction of a compound wherein B is 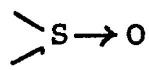 to form a compound wherein B is 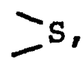,

iii) conversion of a carboxyl group into a non-toxic metabolically labile ester function;

iv) formation of a non-toxic salt function; and

v) removal of any carboxyl blocking and/or N-protecting groups.

6. A process according to claim 5 wherein the compound of formula (II) is (6R,7R)-7-amino-3-(1-pyridiniummethyl)-ceph-3-em-4-carboxylate dihydrochloride.

7. A pharmaceutical composition comprising a compound according to any one of claims 1 to 4 in association with a pharmaceutical carrier or excipient.